# EUROPÄISCHE PATENTSCHRIFT

(11) **EP 3 493 771 B1**
(45) Veröffentlichungstag und Bekanntmachung des Hinweises auf die Patenterteilung: **28.10.2020**
(21) Anmeldenummer: 17755080.3
(22) Anmeldetag: 03.08.2017
(51) Int. Cl.: A61F 2/34, A61F 2/30

(54) **GELENKPFANNE UND GELENKENDOPROTHESE**
ACETABULAR CUP AND PROSTHETIC JOINT
CUVETTE ACÉTABULAIRE ET ENDOPROTHÈSE D'ARTICULATION

(30) Priorität: 03.08.2016 DE 102016114368
(43) Veröffentlichungstag der Anmeldung: 12.06.2019
(73) Patentinhaber: Aesculap AG, 78532 Tuttlingen (DE)
(72) Erfinder: BROGHAMMER, Tanja, 78655 Dunningen (DE); FRITZSCHE, Jörg, 78665 Frittlingen (DE); SCHNEIDER, Jens, 78315 Radolfzell (DE); BISSER, Kai, 78606 Seitingen-Oberflacht (DE)
(74) Vertreter: Hoeger, Stellrecht & Partner Patentanwälte mbB
(86) Internationale Anmeldenummer: PCT/EP2017/069647
(87) Internationale Veröffentlichungsnummer: WO 2018/024825

(56) Entgegenhaltungen:
- EP-A1- 1 163 891
- DE-A1-102005 028 523
- DE-T2- 69 406 786
- FR-A1- 2 826 865
- US-A1- 2013 041 474
- US-A1- 2013 310 945

## Beschreibung

Die vorliegende Erfindung betrifft eine Gelenkpfanne für eine Gelenkendoprothese, insbesondere in Form einer Hüftgelenkpfanne für eine Hüftgelenkendoprothese, welche Gelenkpfanne die Form oder im Wesentlichen die Form eines Ausschnitts einer hohlkugeligen Schale aufweist, wobei die Gelenkpfanne eine Pfannenwand umfasst, in welcher Pfannenwand mindestens eine Durchgangsöffnung ausgebildet ist, welche Durchgangsöffnung mit einem Verschlusselement verschlossen ist, welches Verschlusselement einstückig mit der Pfannenwand ausgebildet ist, wobei mindestens eine Sollbruchstelle zwischen dem Verschlusselement und der Pfannenwand ausgebildet ist, welche mindestens eine Sollbruchstelle irreversibel zerstörbar ist zum Entfernen des Verschlusselements aus der Pfannenwand.

Ferner betrifft die vorliegende Erfindung eine Gelenkendoprothese, insbesondere in Form einer Hüftgelenkendoprothese, umfassend eine Gelenkpfanne und einen mit der Gelenkpfanne kugelgelenkig gekoppelten Schaft, insbesondere in Form eines Femurschafts.

Gelenkpfannen und Gelenkendoprothesen der eingangs beschriebenen Art sind in vielfältiger Weise bekannt, beispielsweise aus der DE 694 06 786 T2 offenbart.

Derartige Gelenkpfannen ermöglichen es einem Operateur, bei Bedarf eines oder mehrere Verschlusselemente aus der Pfannenwand irreversibel herauszutrennen, um die vom jeweiligen Verschlusselement nach dem Herstellen der Gelenkpfanne verschlossene oder im Wesentlichen verschlossene Durchgangsöffnung freizulegen. Durch die freigelegte Durchgangsöffnung kann dann insbesondere eine Befestigungsschraube durchgeführt werden, um die Gelenkpfanne am Knochen zu fixieren.

Das Heraustrennen eines oder mehrerer Verschlusselemente erfolgt üblicherweise während der Implantation der Gelenkendoprothese. So werden nur diejenigen Durchgangsöffnungen durch Herauslösen der Verschlusselemente freigelegt, die tatsächlich benötigt werden.

Ein Problem bei Gelenkpfannen der eingangs beschriebenen Art ist es, eine Durchgangsöffnung wieder zu verschließen, wenn diese letztlich doch nicht erforderlich ist, um eine Knochenschraube durch die Pfannenwand hindurch in den Knochen einzuschrauben, um die Gelenkpfanne am Knochen zu fixieren.

Aus der US 2013/0310945 A1 sind Verschlussstopfen für Durchbrechungen einer Hüftgelenkpfanne bekannt. In der FR 2 826 865 A1 ist eine Gelenkpfanne beschrieben. Eine Gelenkpfanne für eine Hüftgelenkendoprothese ist aus der DE 10 2005 028 523 A1 bekannt. In der EP 1 163 891 A1 ist ein Verschlusselement für ein Endoprothesensystem beschrieben. Abdeckungen von Schraubenlöchern in einer Gelenkpfanne sind in der US 2013/0041474 A1 offenbart.

Es ist daher eine Aufgabe der vorliegenden Erfindung, eine Gelenkpfanne und eine Gelenkendoprothese der eingangs beschriebenen Art so zu verbessern, dass die Durchgangsöffnung nach dem irreversiblen Heraustrennen des Verschlusselements einfach und sicher wieder verschlossen werden kann.

Diese Aufgabe wird bei einer Gelenkpfanne der eingangs beschriebenen Art erfindungsgemäß dadurch gelöst, dass die Gelenkpfanne mindestens eine Verbindungseinrichtung umfasst zum kraft- und/oder formschlüssigen Verbinden des aus der Pfannenwand entfernten Verschlusselements mit der Pfannenwand in einer Verbindungsstellung.

Die vorgeschlagene Weiterbildung einer bekannten Gelenkpfanne ermöglicht es also insbesondere, das Verschlusselement mit der Pfannenwand wieder in definierter Weise zu verbinden, insbesondere kraft- und/oder formschlüssig. Dies ermöglicht es einem Operateur, die Durchgangsöffnung bei Bedarf wieder mit dem Verschlusselement zu verschließen. Insbesondere werden hierfür keine weiteren Verschlusselemente benötigt. Es können diejenigen Verschlusselemente genutzt werden, die nach der Herstellung der Gelenkpfanne zunächst über die mindestens eine Sollbruchstelle mit der Pfannenwand verbunden sind. So ist insbesondere sichergestellt, dass stets die erforderliche Zahl an Verschlusselementen für die Implantation der Gelenkpfanne bereitsteht. Gegebenenfalls können sogar alle freigelegten Durchgangsöffnungen wieder mit den hierfür vorgesehenen Verschlusselementen verschlossen werden. Es können insbesondere mehrere Durchgangsöffnungen vorgesehen sein, die jeweils mit einem Verschlusselement verschlossen sind. Insbesondere können die Durchgangsöffnungen und die Verschlusselemente identisch ausgebildet sein, sodass ein herausgetrenntes Verschlusselement nicht nur zum Verschließen derjenigen Durchgangsöffnung genutzt werden kann, die ursprünglich mit diesem Verschlusselement verschlossen war, sondern auch zum Verschließen einer beliebigen anderen Durchgangsöffnung genutzt werden kann.

Günstigerweise weist die Gelenkpfanne eine kugelige oder im Wesentlichen kugelige Außenfläche und eine hohlkugelige oder im Wesentlichen hohlkugelige Innenfläche auf. So kann zum einen ein Knochen zum Einsetzen der Gelenkpfanne auf einfache Weise mit einem kugeligen Fräser präpariert werden. Zum anderen kann beispielsweise ein Gelenkkopf eines Gelenkschafts direkt in die Gelenkpfanne eingesetzt werden oder ein entsprechender Einsatz zur Ausbildung einer optimalen Gleitpaarung in Verbindung mit dem Gelenkkopf des Gelenkschafts.

Günstig ist es, wenn die mindestens eine Verbindungseinrichtung in Form einer Schraubverbindungseinrichtung, einer Bajonettverbindungseinrichtung, einer Drehklemmverbindungseinrichtung oder einer Schnappverbindungseinrichtung ausgebildet ist. Die genannten Verbindungsarten ermöglichen insbesondere eine schnelle und sichere Verbindung des Verschlusselements mit der Pfannenwand. Eine Verbindung kann insbesondere definiert hergestellt werden, um ein unbeabsichtigtes Lösen des Verschlusselements aus der Durchgangsöffnung in der Pfannenwand zu verhindern.

Vorteilhaft ist es, wenn die mindestens eine Verbindungseinrichtung mindestens ein erstes Verbindungselement und mindestens ein zweites Verbindungselement umfasst, wenn das mindestens eine erste Verbindungselement an der Pfannenwand angeordnet oder ausgebildet ist und wenn das mindestens eine zweite Verbindungselement am mindestens einen Verschlusselement angeordnet oder ausgebildet ist. Es können also ein, zwei, drei, vier oder mehr erste Verbindungselemente oder auch entsprechend ein, zwei, drei, vier oder mehr zweite Verbindungselemente vorgesehen sein, die, nicht zwingend in entsprechender Anzahl, miteinander in der Verbindungsstellung in Eingriff stehen. Insbesondere kann das mindestens eine erste Verbindungselement an der Pfannenwand an oder im Bereich der Durchgangsöffnung angeordnet oder ausgebildet sein. So lässt sich die Gelenkpfanne zudem besonders kompakt ausbilden.

Günstig ist es, wenn das mindestens eine erste Verbindungselement und das mindestens eine zweite Verbindungselement in der Verbindungsstellung kraft- und/oder formschlüssig in Eingriff stehen und in einer Trennstellung, in welcher die mindestens eine Sollbruchstelle irreversibel zerstört ist und das Verschlusselement die mindestens eine Durchgangsöffnung freigibt, außer Eingriff stehen. So lässt sich das die mindestens eine Durchgangsöffnung ursprünglich verschließende Verschlusselement vollständig von der Gelenkpfanne trennen und handhaben und bei Bedarf wieder zum Verschließen der mindestens einen Durchgangsöffnung nutzen.

Auf einfache Weise lässt sich das Verschlusselement zum Verschließen einer Durchgangsöffnung in die Pfannenwand einschrauben, wenn das mindestens eine erste oder zweite Verbindungselement einen Innen- oder Außengewindeabschnitt umfasst. Beispielsweise kann das mindestens eine erste Verbindungselement einen Innengewindeabschnitt umfassen und das mindestens eine zweite Verbindungselement einen korrespondierenden Außengewindeabschnitt oder umgekehrt.

Ferner kann es vorteilhaft sein, wenn das mindestens eine erste oder zweite Verbindungselement in Form eines Vorsprungs oder in Form einer zum Vorsprung korrespondierenden Ausnehmung ausgebildet ist. So kann beispielsweise das mindestens eine erste Verbindungselement in Form eines Vorsprungs und das mindestens eine zweite Verbindungselement in Form einer korrespondierenden Ausnehmung ausgebildet sein oder umgekehrt. Zum Beispiel können zwei, drei oder mehr Vorsprünge und zugehörige Ausnehmungen vorgesehen sein, insbesondere zur Ausbildung einer Bajonettverbindung.

Günstigerweise definiert die mindestens eine Durchgangsöffnung eine Längsachse, welche in radialer Richtung oder im Wesentlichen in radialer Richtung bezogen auf einen Mittelpunkt der Schale verläuft. Dies ermöglicht es insbesondere, Befestigungsschrauben in radialer Richtung zum Befestigen der Gelenkpfanne an einem Knochen ebenfalls in radialer Richtung bezogen auf den Mittelpunkt der Schale einzusetzen. Auf diese Weise stehen Köpfe der Knochenschrauben nur minimal in Richtung auf den Mittelpunkt aus der Pfannenwand vor oder sogar überhaupt nicht. Dies erleichtert gegebenenfalls das Einführen und Verbinden eines Gelenkeinsatzes in die und mit der Gelenkpfanne.

Zur Ausbildung einer Bajonettverbindung oder einer Drehklemmverbindung ist es insbesondere günstig, wenn der Vorsprung vom mindestens einen Verschlusselement bezogen auf die Längsachse in radialer Richtung weisend abstehend oder von einem die Durchgangsöffnung begrenzenden Rand der Pfannenwand in Richtung auf die Längsachse hin vorstehend angeordnet oder ausgebildet ist. So kann das mindestens eine Verschlusselement durch Verdrehen um die Längsachse beispielsweise von der Trennstellung in die Verbindungsstellung überführt werden, wenn ein am Verschlusselement ausgebildeter Vorsprung bei der Verdrehbewegung mit der korrespondieren, am Rand der Durchgangsöffnung ausgebildeten Ausnehmung in Eingriff gebracht wird.

Vorteilhaft ist es, wenn die zum Vorsprung korrespondierende Ausnehmung in von einem die Durchgangsöffnung begrenzenden Rand der Pfannenwand in Richtung auf die Längsachse hin weisend geöffnet oder vom mindestens einen Verschlusselement bezogen auf die Längsachse in radialer Richtung weisend geöffnet angeordnet oder ausgebildet ist. Eine derartig ausgebildete Ausnehmung kann insbesondere mit einem Vorsprung in Eingriff gebracht werden, welcher in entgegengesetzter Richtung weisend vom Verschlusselement oder von der Pfannenwand abstehend ausgebildet ist. Insbesondere kann so auf einfache Weise eine Bajonett- oder Drehklemmverbindung ausgebildet werden.

Vorzugsweise umfasst die Ausnehmung mindestens einen in Richtung auf die Längsachse hin oder in radialer Richtung von der Längsachse weg weisend geöffneten Nutabschnitt. So kann ein korrespondierender Vorsprung in der Ausnehmung einfach und sicher geführt und gegebenenfalls auch geklemmt werden, beispielsweise wenn die Verbindungseinrichtung in Form einer Bajonett- oder Drehklemmverbindung ausgebildet ist.

Ferner kann es vorteilhaft sein, wenn die Ausnehmung mindestens abschnittsweise in Richtung auf einen Mittelpunkt der Schale hin weisend geöffnet ist zum Ausbilden einer Einführöffnung für den Vorsprung oder mindestens einen Teil desselben in einer parallel oder im Wesentlichen parallel zur Längsachse verlaufenden Einführrichtung. Diese Ausgestaltung ermöglicht es insbesondere, das Verschlusselement parallel zur Einführrichtung in die Durchgangsöffnung einzusetzen und dann, wenn der Vorsprung bereits mit der Ausnehmung in Kontakt steht, das Verschlusselement relativ zur Durchgangsöffnung zu verdrehen, wobei der Vorsprung und die Ausnehmung kraft- und/oder formschlüssig miteinander in Eingriff treten, um das Verschlusselement sicher und dauerhaft in der Durchgangsöffnung festzulegen.

Günstigerweise ist der Innen- oder Außengewindeabschnitt am Vorsprung angeordnet oder ausgebildet. So können also mehrere voneinander getrennte, aber insgesamt zusammenwirkende Innen- oder Außengewindeabschnitte vorgesehen sein, die jeweils an einem Vorsprung angeordnet oder ausgebildet sind.

Günstig ist es, wenn der Vorsprung bei unbeschädigter Sollbruchstelle in einer Neutralstellung der mindestens einen Verbindungseinrichtung angeordnet ist, in welcher der Vorsprung in der Einführöffnung oder im Bereich der Einführöffnung angeordnet ist. Dies bedeutet insbesondere, dass das Verschlusselement mit der Pfannenwand nicht kraft- und/oder formschlüssig in Eingriff stehen würde, wenn die Sollbruchstelle durchtrennt wäre. Dies ermöglicht es insbesondere, nicht herausgetrennte Verschlusselemente zwar durch Durchbrechen der mindestens einen Sollbruchstelle von der Pfannenwand zu lösen, diese dann aber sofort von der Neutralstellung, die die Verschlusselemente dann einnehmen, in die Verbindungsstellung zu überführen, beispielsweise durch Einschrauben oder Verdrehen in die beziehungsweise relativ zur Pfannenwand.

Günstigerweise steht das Verschlusselement in der Neutralstellung bei irreversibel zerstörter Sollbruchstelle mit der Pfannenwand weder kraft- noch formschlüssig in Eingriff. Diese Ausgestaltung ermöglicht insbesondere ein einfaches und leichtes Ausbrechen des Verschlusselements, da lediglich die Sollbruchstelle zerstört werden muss, nicht jedoch eine kraft- und/oder formschlüssige Verbindung zwischen dem Verschlusselement und der Pfannenwand gelöst werden muss.

Vorteilhafterweise weist die mindestens eine Sollbruchstelle eine Wandstärke auf, welche deutlich kleiner ist als eine Dicke der Pfannenwand. So kann ganz gezielt und auf einfache Weise das Verschlusselement aus der Pfannenwand herausgetrennt werden. Bevorzugt wird dadurch lediglich die Sollbruchstelle irreversibel zerstört, nicht jedoch die Pfannenwand beschädigt.

Vorteilhaft ist es, wenn die Wandstärke weniger als das 0,2-fache der Dicke beträgt. Vorzugsweise beträgt sie weniger als das 0,1-fache der Dicke. So lässt sich das Verschlusselement aus der Pfannenwand einfach und schnell herauslösen.

Ferner ist es vorteilhaft, wenn in der Pfannenwand mindestens ein Kanal ausgebildet ist, welcher die Pfannenwand mindestens teilweise durchsetzt und ein erstes offenes Ende und ein zweites offenes Ende aufweist, und wenn das erste offene Ende im Bereich des ersten oder zweiten Verbindungselements angeordnet oder ausgebildet ist und wenn das zweite offene Ende die Außenfläche oder die Innenfläche der Pfannenwand durchsetzt. Eine solche Ausgestaltung der Pfannenwand mit mindestens einem Kanal ermöglicht es auf einfache Weise, die Gelenkpfanne einstückig, insbesondere monolithisch, durch Sintern, beispielsweise Lasersintern, aus einem pulverförmigen Ausgangsmaterial auszubilden. Den mindestens einen Kanal vorzusehen ermöglicht es insbesondere, überschüssiges Ausgangsmaterial, welches beispielsweise im Bereich zwischen dem mindestens einen ersten und dem mindestens einen zweiten Verbindungselement nach dem Sintern verbleibt, einfach und sicher zu entfernen. Der mindestens eine Kanal kann daher auch optional als Pulverkanal bezeichnet werden. So lassen sich sogar teilweise ineinander greifende Strukturen des mindestens einen ersten und des mindestens einen zweiten Verbindungselements, insbesondere mit mehreren Hinterschneidungen, miteinander beispielsweise in einem einzigen Fertigungsschritt herstellen, allerdings ohne die Gefahr, dass das Pulver teilweise in der Pfannenwand verbleibt und erst im Verlauf einer Implantation der Gelenkpfanne heraustreten kann. Dadurch, dass das zweite offene Ende die Außenfläche oder die Innenfläche der Pfannenwand durchsetzt, wird eine Mündung des mindestens einen Kanals ausgebildet, welcher insbesondere das Herausfallen von pulverförmigem Ausgangsmaterial durch die Innenfläche oder durch die Außenfläche der Pfannenwand ermöglicht.

Um im Bereich zwischen dem mindestens einen ersten und dem mindestens einen zweiten Verbindungselement verbliebenes pulverförmiges Ausgangsmaterial einfach und sicher entfernen zu können, ist es günstig, wenn ein Durchmesser des mindestens einen Kanals mindestens etwa dem 5-fachen, insbesondere dem 10-fachen des mittleren Durchmessers der Pulverteilchen entspricht, aus denen die Gelenkpfanne durch ein generatives Fertigungsverfahren ausgebildet ist. Vorzugsweise ist der Durchmesser des mindestens einen Kanals mindestens etwa fünf Mal größer als der größte Durchmesser der Pulverteilchen aus denen die Gelenkpfanne durch ein generatives Fertigungsverfahren ausgebildet ist. So kann insbesondere sichergestellt werden, dass das pulverförmige Ausgangsmaterial zur Ausbildung der Gelenkpfanne den mindestens einen Kanal nicht verstopfen und aus diesem in gewünschter Weise austreten kann.

Um möglichst effizient nicht verfestigtes pulverförmiges Ausgangsmaterial aus einem Bereich zwischen dem mindestens einen ersten und dem mindestens einen zweiten Verbindungselement zu entfernen, ist es vorteilhaft, wenn die Gelenkpfanne eine Mehrzahl von Kanälen umfasst. Beispielsweise können jeder Paarung eines ersten und eines zweiten Verbindungselements ein, zwei oder drei Kanäle in der beschriebenen Weise zugeordnet sein.

Ferner ist es vorteilhaft, wenn das mindestens eine durch Zerstören der mindestens einen Sollbruchstelle aus der Pfannenwand herausgetrennte Verschlusselement durch Einschrauben um eine von der Durchgangsöffnung definierte Längsachse oder eine Eindrehbewegung um die Längsachse von der Trennstellung in die Verbindungsstellung überführbar ist. Eine Gelenkpfanne, die eine derartige Verbindung des mindestens einen Verschlusselements mit der Pfannenwand zum Verschließen der mindestens einen Durchgangsöffnung vorsieht, ermöglicht es insbesondere, die Sollbruchstelle zu zerstören und direkt anschließend das Verschlusselement in die Verbindungsstellung zu bringen, beispielsweise durch Eindrehen oder Einschrauben.

Um die Handhabung der Gelenkpfanne weiter zu verbessern, ist es günstig, wenn das mindestens eine Verschlusselement mindestens eine Werkzeugelementaufnahme umfasst. Die mindestens eine Werkzeugelementaufnahme kann insbesondere dazu dienen, mit einem Ausbrechwerkzeug beziehungsweise einem Verbindungswerkzeug in Eingriff gebracht zu werden, um das mindestens eine Verschlusselement von der Pfannenwand durch Zerstören der mindestens einen Sollbruchstelle zu lösen und gegebenenfalls wieder mit der Pfannenwand in Eingriff zu bringen, um die mindestens eine Durchgangsöffnung zu verschließen. Die mindestens eine Werkzeugelementaufnahme kann insbesondere in Form einer Ausnehmung oder in Form eines Vorsprungs ausgebildet sein. Beispielsweise kann sie in Form einer Ausnehmung mit einem vieleckigen Querschnitt oder in Form eines Innenvielrunds ausgebildet sein.

Günstigerweise ist die mindestens eine Werkzeugelementaufnahme in Richtung auf den Mittelpunkt der Schale hin geöffnet. So kann insbesondere ein Verschlusselement noch mit der Gelenkpfanne zum Verschließen der mindestens einen Durchgangsöffnung in Eingriff gebracht werden, wenn die Gelenkpfanne bereits am Knochen fixiert ist. Nicht benötigte Durchgangsöffnungen lassen sich so noch nach der Implantation auf einfache Weise verschließen, da die Werkzeugelementaufnahme bei bestimmungsgemäßer Implantation vom Knochen weg weisend geöffnet und für ein entsprechendes Werkzeug zugänglich ist.

Für die Handhabung der Gelenkpfanne ist es besonders vorteilhaft, wenn sie einstückig ausgebildet ist. Insbesondere kann sie monolithisch ausgebildet sein. So ist es insbesondere möglich, die Gelenkpfanne ohne daran beweglich angeordnete Teile auszubilden. Zudem kann so beispielsweise auch sichergestellt werden, dass das mindestens eine Verschlusselement sich nicht in unerwünschter Weise von der Gelenkpfanne lösen kann.

Vorteilhaft ist es, wenn die Gelenkpfanne durch ein generatives Fertigungsverfahren ausgebildet ist. So lassen sich praktisch beliebige Hinterschnitte und ineinander greifende Elemente der Gelenkpfanne ohne größere Schwierigkeiten ausbilden. Insbesondere sind so also komplexe Formgebungen im Bereich miteinander zusammenwirkender erster und zweiter Verbindungselemente möglich.

Vorzugsweise ist die Gelenkpfanne durch ein Pulverbettverfahren, durch ein Freiformverfahren, durch ein Flüssigmaterialverfahren oder durch ein anderes Schichtaufbauverfahren ausgebildet. So lassen sich insbesondere Gelenkpfannen aus einem Metall oder einem Metallgemisch oder aus einem Kunststoff oder einem Kunststoffgemisch ausbilden.

Auf einfache Weise kann die Gelenkpfanne hergestellt werden, wenn sie durch selektives Laserschmelzen, durch selektives Lasersintern, durch selektives Wärmesintern, durch Verfestigen von Pulvermaterial mittels Binder, durch Elektronenstrahlschmelzen, durch Auftragsschweißen, durch ein Metall-Pulver-Auftragsverfahren, durch Kaltgasspritzen, durch Stereolithografie, durch ein Digital-Light-Processing zum Belichten einzusetzendes Verfahren, durch schichtweises Laminieren, durch 3D-Siebdruck von Metallen oder durch lichtgesteuerte elektrophoretische Abscheidung ausgebildet ist. In Abhängigkeit eines zur Herstellung der Gelenkpfanne gewünschten Materials lässt sich so das individuell vorteilhafteste Herstellungsverfahren für den jeweiligen Typ der Gelenkpfanne auswählen, um eine optimale Stabilität und Festigkeit der Gelenkpfanne zu erhalten.

Günstigerweise ist die Gelenkpfanne aus einem Metall und/oder aus einem Kunststoff und/oder aus einem Metall-Kunststoff-Gemisch ausgebildet. Insbesondere lassen sich durch ein generatives Fertigungsverfahren Metall-Kunststoff-Gemische zur Ausbildung beispielsweise der Pfannenwand einsetzen. Es ist auch denkbar, einzelne Bereiche der Gelenkpfanne einerseits aus einem Metall oder einem Metallgemisch und andererseits aus einem Kunststoff oder einem Kunststoffgemisch auszubilden.

Vorzugsweise ist oder enthält das Metall Titan. So lassen sich insbesondere gut körperverträgliche Gelenkpfannen ausbilden.

Ferner kann es vorteilhaft sein, wenn der Kunststoff Polyetheretherketon ist oder enthält. Dieser weist insbesondere eine hohe Körperverträglichkeit auf.

Die eingangs gestellte Aufgabe wird ferner bei einer Kniegelenkendoprothese der eingangs beschriebenen Art erfindungsgemäß dadurch gelöst, dass die Gelenkpfanne in Form einer der oben beschriebenen vorteilhaften Gelenkpfannen ausgebildet ist.

Die Gelenkendoprothese weist dann ebenfalls die oben im Zusammenhang mit bevorzugten Ausführungsformen beschriebenen Vorteile auf. Die Gelenkendoprothese kann optional auch in Form einer Schultergelenkendoprothese oder eines Zwischenwirbelimplantats ausgebildet sein.

Vorteilhaft ist es, wenn der Schaft einen kugelförmigen oder im Wesentlichen kugelförmigen Gelenkkopf umfasst und wenn die Gelenkpfanne einen Gelenkeinsatz mit einer zum Gelenkkopf korrespondierenden Gelenkkopfaufnahme umfasst. Auf diese Weise lässt sich ein optimiertes Kugelgelenk mit einer Gleitpaarung ausbilden, die einen möglichst geringen Verschleiß aufweist.

Die nachfolgende Beschreibung bevorzugter Ausführungsformen der Erfindung dient im Zusammenhang mit den Zeichnungen der näheren Erläuterung. Es zeigen:
- Figur 1A:: eine perspektivische Ansicht einer Gelenkpfanne von außen;
- Figur 1B:: eine perspektivische Ansicht der Gelenkpfanne aus Figur 1 von innen;
- Figur 2A:: eine schematische, vergrößerte perspektivische Ansicht des Bereichs A in Figur 1B;
- Figur 2B:: eine Schnittansicht längs Linie 2B-2B in Figur 2A;
- Figur 3:: eine Schnittansicht ähnlich Figur 2B eines weiteren Ausführungsbeispiels einer Gelenkpfanne;
- Figur 4:: eine Schnittansicht eines weiteren Ausführungsbeispiels einer Gelenkpfanne im Bereich einer mit einem Verschlusselement verschlossenen Durchgangsöffnung;
- Figur 5:: eine Schnittansicht längs Linie 4-4 in Figur 4;
- Figur 6:: eine perspektivische schematische Darstellung eines weiteren Ausführungsbeispiels eines Verschlusselements mit Teilausschnittsvergrößerung ;
- Figur 7:: eine schematische perspektivische Darstellung eines weiteren Ausführungsbeispiels eines Verschlusselements;
- Figur 8:: eine schematische Darstellung eines weiteren Ausführungsbeispiels eines Verschlusselements; und
- Figur 9:: eine schematische Schnittansicht ähnlich Figur 3 eines weiteren Ausführungsbeispiels einer Gelenkpfanne im Bereich einer mit einem Verschlusselement verschlossenen Durchgangsöffnung.

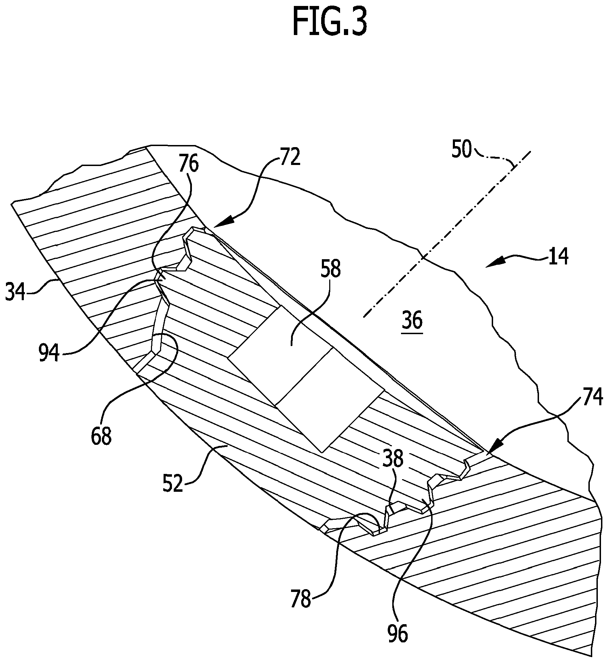

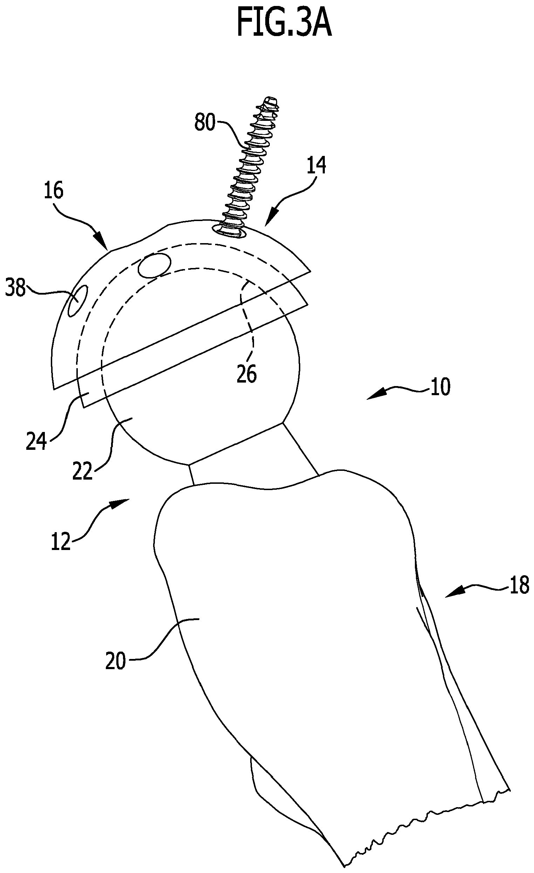

In Figur 3A ist schematisch eine Gelenkendoprothese 10 in Form einer Hüftgelenkendoprothese 12 schematisch dargestellt.

Die Gelenkendoprothese 10 umfasst eine Gelenkpfanne 14 in Form einer Hüftgelenkpfanne 16 und einen mit der Gelenkpfanne 14 kugelgelenkig gekoppelten Schaft 18 in Form eines Femurschafts 20.

Der Schaft 18 umfasst einen kugelförmigen oder im Wesentlichen kugelförmigen Gelenkkopf 22, welcher mit einem in die Gelenkpfanne 14 eingesetzten Gelenkeinsatz 24 eine Gleitpaarung bildet. Zum Aufnehmen des Gelenkkopfs 22 weist der Gelenkeinsatz 24 eine zum Gelenkkopf 22 korrespondierende Gelenkkopfaufnahme 26 auf.

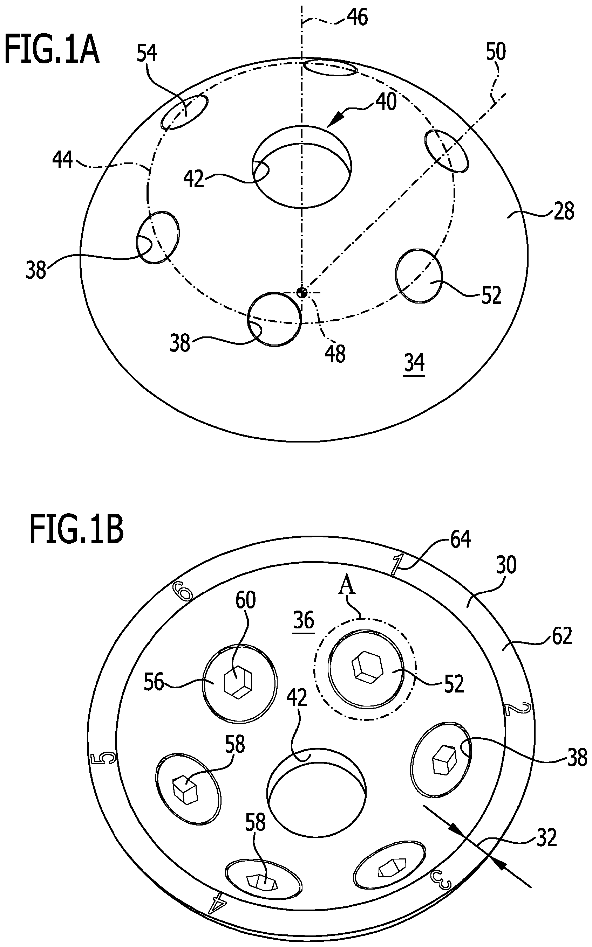

Die Gelenkpfanne 14 ist in den Figuren 1A und 1B detaillierter dargestellt.

Die Gelenkpfanne 14 ist in Form oder im Wesentlichen in Form eines Ausschnitts einer hohlkugeligen Schale 28 ausgebildet. Die Schale 28 umfasst eine Pfannenwand 30 mit einer Dicke 32 und definiert eine kugelige Außenfläche 34 und eine hohlkugelige oder im Wesentlichen hohlkugelige Innenfläche 36. Insbesondere kann die Schale 28 die Form einer halben Hohlkugel aufweisen.

In der Pfannenwand 30 sind mehrere Durchgangsöffnungen 38 ausgebildet, die eine zentrale in einem Scheitelbereich 40 der Schale 28 ausgebildete Öffnung 42 umgebend angeordnet sind, und zwar gleichmäßig auf einem Kreis 44, welcher eine Rotationsachse 46 konzentrisch umgibt. Die Rotationsachse 46 bildet eine Symmetrieachse der Schale 28, bezüglich derer die Schale 28 rotationssymmetrisch ausgebildet ist.

Die Durchgangsöffnungen 38 sind über den Umfang des Kreises 44 gleichmäßig verteilt angeordnet. Die Durchgangsöffnungen 38 sind in radialer Richtung weisend von einem Mittelpunkt 48 der Gelenkpfanne 44 ausgehend geöffnet und definieren jeweils eine Längsachse 50, so dass sich alle Längsachsen 50 im Mittelpunkt 48 mit der Rotationsachse 46 schneiden.

Bei dem in den Figuren 1A und 1B schematisch dargestellten Ausführungsbeispiel der Gelenkpfanne 14 sind insgesamt sechs Durchgangsöffnungen 38 vorgesehen, die jeweils mit einem Verschlusselement 52 verschlossen sind.

Die Verschlusselemente 52 sind einstückig mit der Pfannenwand 30 ausgebildet. Eine vom Mittelpunkt 48 weg weisende Außenfläche 54 des Verschlusselements 52 kann insbesondere eine Krümmung aufweisen, die einer Krümmung der Außenfläche 34 entspricht, sodass die Außenfläche 54 quasi einen Teil der Außenfläche 34 bildet.

Eine in Richtung auf den Mittelpunkt 48 hin weisende Innenfläche 56 des Verschlusselements 52 bildet in ähnlicher Weise einen Teil der Innenfläche 36.

Das Verschlusselement 52 weist ferner eine Werkzeugelementaufnahme 58 auf, welche in Richtung auf den Mittelpunkt 48 der Schale 28 hin weisend geöffnet ist. Die Werkzeugelementaufnahme 58 ist in Figur 1B beispielhaft als Innensechskant 60 ausgebildet. Alternativ sind auch andere vieleckige Innenquerschnitte möglich oder beispielsweise auch ein Innenvielrund.

Die zentrale Öffnung 42 im Scheitelbereich ist nicht verschlossen. Sie dient insbesondere zum Koppeln der Gelenkpfanne 14 mit dem Gelenkeinsatz 24.

In Figur 1B ist ferner auf einem die Innenfläche 36 mit der Außenfläche 34 verbindenden Rand 62 eine Nummerierung 64 vorgesehen, gemäß der die sechs Durchgangsöffnungen 38 in der in Figur 1B dargestellten Ansicht im Uhrzeigersinn von 1 bis 6 durchnummeriert sind.

Die Gelenkpfanne 14 ist einstückig ausgebildet, insbesondere monolithisch. Dies bedeutet, dass die Verschlusselemente 52 mit der Schale 28 einstückig ausgebildet sind.

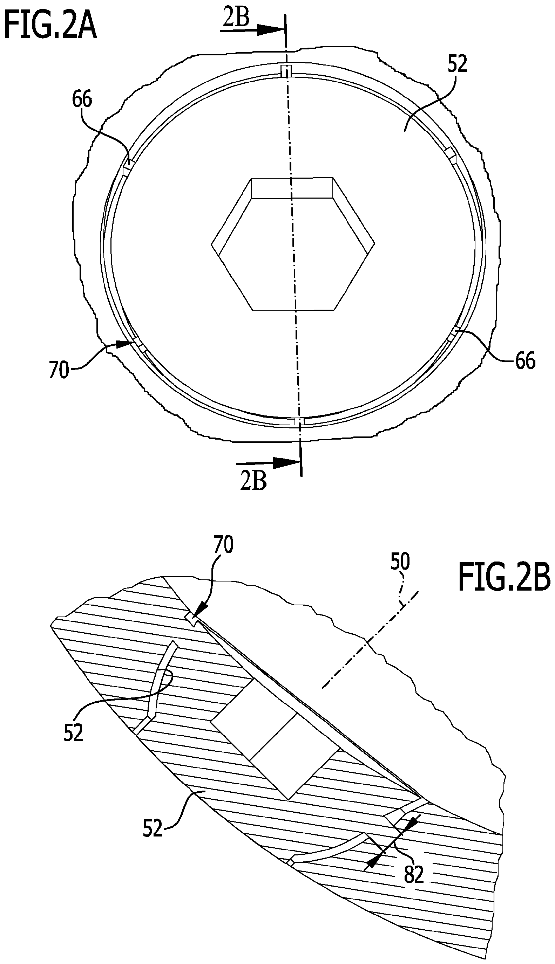

Wie beispielhaft in Figur 2A dargestellt, ist das Verschlusselement 52 über sechs in radialer Richtung bezogen auf die Längsachse verlaufende Stege 66 mit einem in Richtung auf die Längsachse 50 hin weisenden und diese umgebenden Rand 68 verbunden.

Die Stege 66 bilden Sollbruchstellen 70, die irreversibel zerstört werden können, beispielsweise durch Brechen, um das mit der Schale 28 einstückig ausgebildete Verschlusselement 52 aus der Durchgangsöffnung 38 herauszulösen. Dies kann beispielweise dadurch erreicht werden, dass mit einem zur Werkzeugelementaufnahme 58 korrespondierenden Werkzeug in diese eingegriffen und das Verschlusselement 52 um die Längsachse 50 verdreht und/oder verkippt wird.

In Figur 3 ist ein weiteres Ausführungsbeispiel einer Gelenkpfanne 14 schematisch dargestellt. Der Übersichtlichkeit wegen sind hier identische oder ähnliche Elemente mit denselben Bezugszeichen bezeichnet. Dies gilt entsprechend auch für weitere nachfolgend beschriebene Ausführungsbeispiele von Gelenkpfannen 14.

Das Verschlusselement 52 kann ebenfalls über in Figur 3 nicht dargestellte Sollbruchstellen 70 aus der Pfannenwand 30 herausgetrennt werden.

Allerdings ermöglicht es das Verschlusselement 52, wieder kraft- und/oder formschlüssig mit der Pfannenwand 30 verbunden zu werden. Hierfür dient eine Verbindungseinrichtung 72 in Form einer Schraubverbindungseinrichtung 74. Nach dem Lösen des Verschlusselements 52 wie oben beschrieben durch Eingreifen eines Schraubwerkzeugs in die Werkzeugelementaufnahme 58 und Verdrehen des Verschlusselements 52 relativ zur Pfannenwand 30 kann das Verschlusselement 52 durch eine Einschraubbewegung um die Längsachse 50 wieder in die Durchgangsöffnung 38 in der Pfannenwand 30 eingesetzt und mit dieser kraft- und/oder formschlüssig verbunden werden. Hierzu ist am Verschlusselement 52 ein Außengewindeabschnitt 76 mit ein bis drei Gewindegängen und im Rand 68 im Bereich der Durchgangsöffnung 38 ein Innengewindeabschnitt 78 mit ebenfalls etwa ein bis drei Gewindegängen ausgebildet, und zwar korrespondierend zum Außengewindeabschnitt 76.

Die Verschlusselemente 52 können insbesondere entfernt werden, um die Durchgangsöffnung 38 freizugeben, durch die hindurch ein Befestigungselement 80 teilweise hindurchgeführt werden kann, beispielsweise ein Schaft einer Knochenschraube, um die Gelenkpfanne 14 in einem Knochen eines Patienten zu verankern.

Falls bei der Implantation der Gelenkpfanne 14 mehr Durchgangsöffnungen 38 durch Entfernen der Verschlusselemente 52 freigelegt wurden, können diese wieder durch Einschrauben um die Längsachse 50 von einer Trennstellung, in welcher die mindestens eine Sollbruchstelle 70 irreversibel zerstört ist und das Verschlusselement 52 die jeweilige Durchgangsöffnung 38 freigibt, in eine Verbindungsstellung überführt werden, in welcher das Verschlusselement 52 und die Pfannenwand 30 kraft- und/oder formschlüssig in Eingriff stehen. Figur 3 zeigt diese Verbindungsstellung schematisch.

Eine Wandstärke 82 der Sollbruchstelle 70 ist vorzugsweise deutlich kleiner als die Dicke 32 der Pfannenwand. Insbesondere beträgt die Wandstärke 82 weniger als 30 % der Dicke 32. In Abhängigkeit einer Dicke 32 der Pfannenwand 30 kann die Wandstärke 82 sogar weniger als 10 % der Dicke betragen.

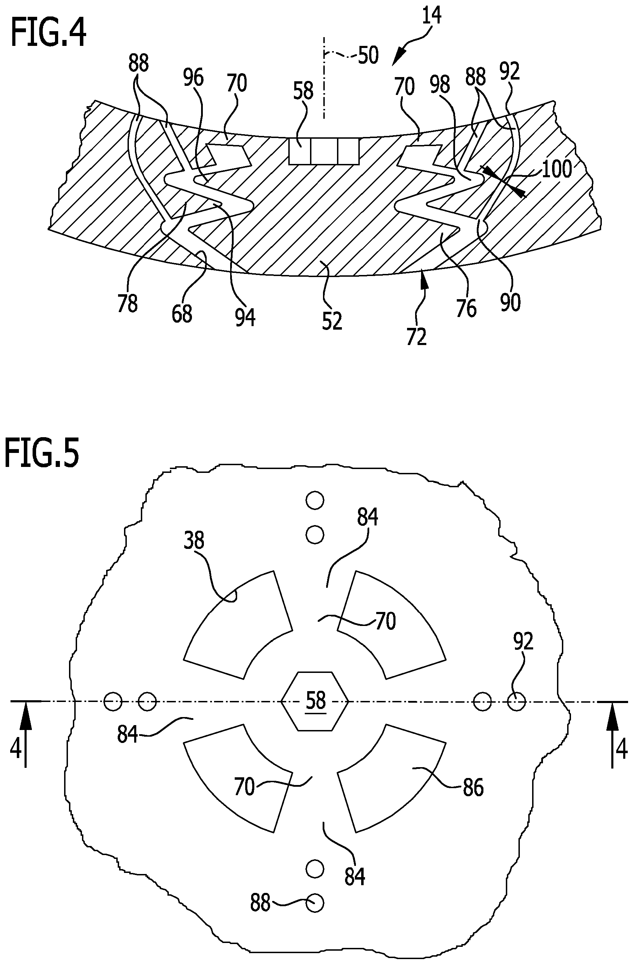

In den Figuren 4 und 5 ist ein weiteres Ausführungsbeispiel einer Gelenkpfanne 14 ausschnittsweise dargestellt. Auch hier weist das Verschlusselement 52 wiederum einen Außengewindeabschnitt 76 auf, welcher im Zusammenwirken mit einem Innengewindeabschnitt 78, welcher am Rand 68 der Durchgangsöffnung 38 ausgebildet ist, eine Verbindungseinrichtung 72 ausbildet.

In Figur 5 ist zu erkennen, dass der Außengewindeabschnitt 76 vier in Umfangsrichtung voneinander getrennte Segmente umfassen kann, die an in radialer Richtung auf die Längsachse 50 vom Rand 68 vorstehenden Vorsprüngen 84 ausgebildet sind. Insgesamt sind vier Vorsprünge 84 ausgebildet, die gleichmäßig über den Umfang des Rands 68 verteilt sind und sich über einen Winkelbereich von etwa 30° erstrecken. Dadurch wird zwischen zwei Vorsprüngen 84 jeweils eine Öffnung 86 ausgebildet, die sich über einen Umfangswinkel von etwa 60° erstreckt.

Um die Gelenkpfanne 14 insbesondere in einem generativen Fertigungsverfahren herstellen zu können, können optional in der Pfannenwand 30 ein oder mehrere Kanäle 88 ausgebildet sein. Diese sind beispielhaft in Figur 4 eingezeichnet.

Jeder Kanal 88 durchsetzt die Pfannenwand 30 mindestens teilweise und weist ein erstes offenes Ende 90 und ein zweites offenes Ende 92 auf. Das erste offene Ende 90 ist im Bereich zwischen dem Innengewindeabschnitt 78, welcher ein erstes Verbindungselement 94 der Verbindungseinrichtung 72 bildet, und dem Außengewindeabschnitt 76, welcher ein zweites Verbindungselement 96 der Verbindungseinrichtung 72 bildet, angeordnet oder ausgebildet. Das zweite offene Ende 92 durchsetzt die Innenfläche 36 der Pfannenwand 30. Alternativ kann das zweite offene Ende 92 auch die Außenfläche 34 der Pfannenwand 30 durchsetzen.

Die Kanäle 88 dienen insbesondere dazu, pulverförmiges Ausgangsmaterial, aus welchem die Gelenkpfanne 14 beispielsweise durch selektives Laserschmelzen oder Lasersintern ausgebildet wird, welches zwischen den Verbindungselementen 94 und 96 nicht verfestigt wird, aus einem zwischen den Verbindungselementen 94 und 96 ausgebildeten Spalt 98 wieder zu entfernen.

Ein Durchmesser 100 des Kanals 88 beträgt mindestens etwa das 5-fache, vorzugsweise das 10-fache eines mittleren Durchmessers von Pulverteilchen, aus denen die Gelenkpfanne 14 durch ein generatives Fertigungsverfahren ausgebildet ist. Vorzugsweise ist sichergestellt, dass der Durchmesser 100 in jedem Fall größer ist als ein maximaler Durchmesser der Pulverteilchen.

Auch bei dem in den Figuren 4 und 5 dargestellten Ausführungsbeispiel der Gelenkpfanne 14 kann das Verschlusselement 52 durch Zerstören der Sollbruchstelle 70 aus der Pfannenwand 30 herausgetrennt werden. Wird die Durchgangsöffnung 38 nicht zum Einsetzen eines Befestigungselements benötigt, kann es wie bereits oben beschrieben durch Eindrehen um die Längsachse 50 wieder in die Durchgangsöffnung 38 der Pfannenwand 30 eingeschraubt werden, um eine kraft- und/oder formschlüssige Verbindung mit dieser einzugehen.

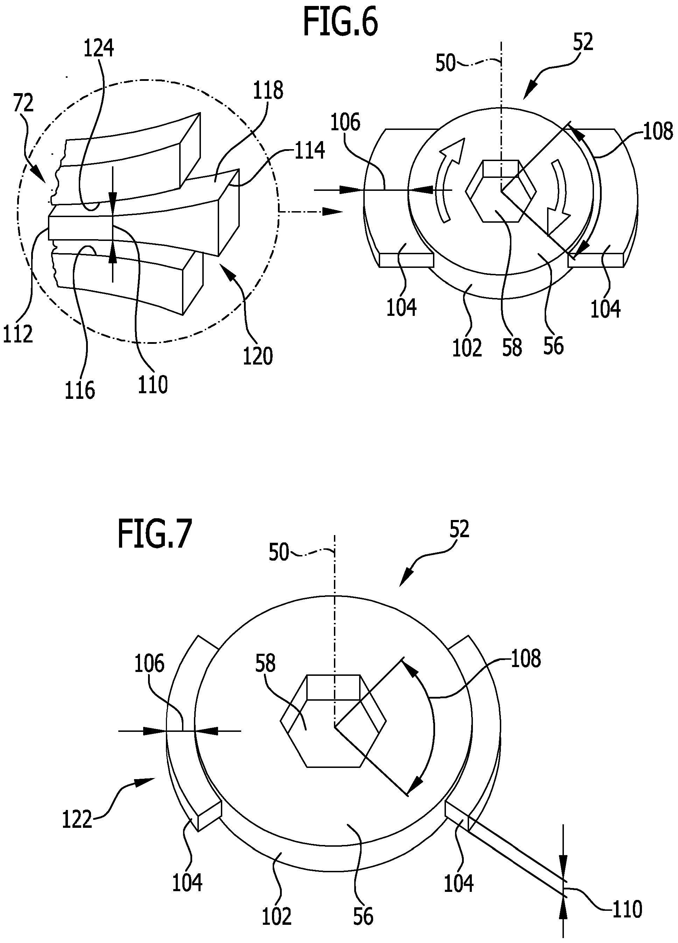

In den Figuren 6 bis 8 sind weitere Ausführungsbeispiele von Verschlusselementen 52 dargestellt. Sie sind ebenfalls im Wesentlichen scheibenförmig ausgebildet und weisen von ihrer Innenfläche 56 weg weisend eine Werkzeugelementaufnahme 58 auf.

An einem das Verschlusselement 52 umlaufend umgebenden Umfangsrand 102 sind in diametraler Richtung voneinander weg weisend zwei abstehende Vorsprünge 104 ausgebildet. Diese unterscheiden sich bei den verschiedenen Ausführungsbeispielen im Wesentlichen durch eine Höhe 106 in radialer Richtung ausgehend von der Längsachse 50 sowie einen Umfangswinkel 108 voneinander, über welchen sich der Vorsprung 104 in Umfangsrichtung bezogen auf die Längsachse 20 erstreckt.

Ferner kann sich eine Dicke 110 der Vorsprünge 104 parallel zur Längsachse 50 in Abhängigkeit des Umfangswinkels 108 ändern. In der Ausschnittsansicht in Figur 6 ist schematisch dargestellt, wie diese Dicke 110 ausgehend von einem ersten Ende 112 zu einem zweiten Ende 114 in Umfangsrichtung kontinuierlich zunimmt. So kann durch Drehen des Verschlusselements 50 um die Längsachse, wenn der Vorsprung 104 in eine korrespondierende, in den Figuren 6 bis 8 nicht dargestellte Ausnehmung 116, die im Bereich des Rand 68 der Gelenkpfanne 14 ausgebildet ist, das Verschlusselement 52 mit der Pfannenwand 30 verklemmen. Dies kann insbesondere erreicht werden, wenn aufeinander zu weisende Innenflächen der Ausnehmung 116 an voneinander weg weisenden Seitenflächen 118 des Vorsprungs 104 aufgleiten bis eine Verdrehung nicht mehr möglich ist. So kann wahlweise eine Verbindungseinrichtung 72 in Form einer Drehklemmverbindungseinrichtung 120 oder, wenn eine Dicke 110 unabhängig vom Umfangswinkel 108 konstant bleibt, eine Bajonettverbindungseinrichtung 122 ausgebildet werden.

Die zum Vorsprung 104 korrespondierende Ausnehmung 116 ist im Rand 68 in Richtung auf die Längsachse 50 hin weisend geöffnet ausgebildet. Umgekehrt kann die Ausnehmung 116 auch am Verschlusselement 52 ausgebildet sein und bezogen auf die Längsachse 50 in radialer Richtung weg weisend geöffnet angeordnet oder ausgebildet sein. Ein entsprechender Vorsprung 104 ist dann vom Rand 68 abstehend ausgebildet.

Die Ausnehmung 116 kann insbesondere in Form einer in Umfangsrichtung bezogen auf die Längsachse 50 verlaufenden Nut 124 ausgebildet sein.

Die Ausnehmung 116 kann zudem mindestens abschnittsweise in Richtung auf den Mittelpunkt 48 der Schale 28 hin weisend geöffnet sein zum Ausbilden einer Einführöffnung für den Vorsprung 104 oder mindestens einen Teil desselben in einer parallel oder im Wesentlichen parallel zur Längsachse 50 verlaufenden Einführrichtung.

Insbesondere kann der Vorsprung 104 bei unbeschädigter Sollbruchstelle 70 in einer Neutralstellung der mindestens einen Verbindungseinrichtung 72 angeordnet sein, in welcher der Vorsprung 104 in der Einführöffnung oder im Bereich der Einführöffnung der Ausnehmung 116 angeordnet ist.

Dies ermöglicht es insbesondere, dass das Verschlusselement 52 in einer Neutralstellung auch bei irreversibel zerstörter Sollbruchstelle 70 mit der Pfannenwand 30 weder kraft- noch formschlüssig in Eingriff steht.

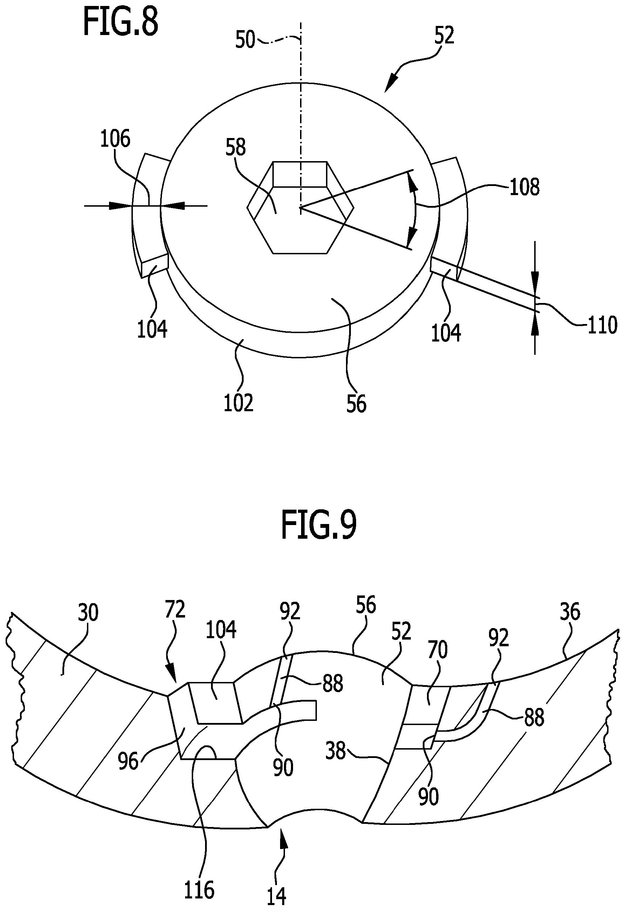

In Figur 9 ist ein weiteres Ausführungsbeispiel einer Gelenkpfanne 14 schematisch dargestellt. Bei diesem ist das Verschlusselement 52 wiederum einstückig über eine Sollbruchstelle mit der Pfannenwand 30 verbunden.

Kanäle 88 sind sowohl an der Pfannenwand 30 als auch am Verschlusselement 52 ausgebildet und münden mit ihrem zweiten Ende 92 an der Innenfläche 36 beziehungsweise der Innenfläche 56.

Erste Enden 90 der Kanäle 88 münden dagegen in einen Bereich zwischen der ein erstes Verbindungselement 94 bildenden Ausnehmung 116 und dem ein zweites Verbindungselement 96 der Verbindungseinrichtung 72 bildenden Vorsprung 104. Hier ist die Verbindungseinrichtung 72 bei unzerstörter Sollbruchstelle 70 in einer Neutralstellung angeordnet. Aus dieser könnte das Verschlusselement 52 ungehindert aus der Durchgangsöffnung 38 herausfallen, wenn die Sollbruchstelle 70 beziehungsweise die mehreren Sollbruchstellen 70 irreversibel zerstört sind.

Durch entsprechendes Verdrehen des Verschlusselements 52 in der Durchgangsöffnung 38 um die Längsachse 50 kann es zu einer Verklemmung zwischen den Vorsprüngen 104 am Verschlusselement 52 und korrespondierenden Ausnehmungen 116 am Rand 68 der Gelenkpfanne 30 kommen. In der so definierten Verbindungsstellung ist das Verschlusselement 52 kraft- und optional auch formschlüssig in der Durchgangsöffnung 38 diese verschließend angeordnet.

Wie bereits erwähnt ist die Gelenkpfanne 14 bevorzugt einstückig ausgebildet. Insbesondere ist sie monolithisch ausgebildet, also aus einem Stück.

Sie kann beispielsweise durch ein generatives Fertigungsverfahren hergestellt werden. Beispielsweise kann es sich dabei ein Pulverbettverfahren, ein Freiformverfahren oder ein Flüssigmaterialverfahren handeln. Auch andere Schichtaufbauverfahren sind denkbar zur Ausbildung der Gelenkpfanne 14.

Wie eingangs beschrieben kann außer selektivem Laserschmelzen oder Lasersintern auch selektives Wärmesintern zum Einsatz kommen. Die Gelenkpfanne 14 kann auch durch Verfestigen von Pulvermaterial mittels eines Binders oder durch Elektronenstrahlschmelzen eines pulverförmigen Ausgangsmaterials ausbildet sein. Auch Auftragsschweißen oder Metall-Pulver-Auftragsverfahren sind geeignet zur Ausbildung der beschriebenen Gelenkpfanne 14.

Die Gelenkpfanne 14 kann insbesondere aus einem Metall und/oder aus einem Kunststoff ausgebildet sein. Vorzugsweise ist das Metall Titan oder enthält Titan. Der Kunststoff kann insbesondere Polyetheretherketon sein oder enthalten.

### Bezugszeichenliste

- 10: Gelenkendoprothese
- 12: Hüftgelenkendoprothese
- 14: Gelenkpfanne
- 16: Hüftgelenkpfanne
- 18: Schaft
- 20: Femurschaft
- 22: Gelenkkopf
- 24: Gelenkeinsatz
- 26: Gelenkkopfaufnahme
- 28: Schale
- 30: Pfannenwand
- 32: Dicke
- 34: Außenfläche
- 36: Innenfläche
- 38: Durchgangsöffnung
- 40: Scheitelbereich
- 42: Öffnung
- 44: Kreis
- 46: Rotationsachse
- 48: Mittelpunkt
- 50: Längsachse
- 52: Verschlusselement
- 54: Außenfläche
- 56: Innenfläche
- 58: Werkzeugelementaufnahme
- 60: Innensechskant
- 62: Rand
- 64: Nummerierung
- 66: Steg
- 68: Rand
- 70: Sollbruchstelle
- 72: Verbindungseinrichtung
- 74: Schraubverbindungseinrichtung
- 76: Außengewindeabschnitt
- 78: Innengewindeabschnitt
- 80: Befestigungselement
- 82: Wandstärke
- 84: Vorsprung
- 86: Öffnung
- 88: Kanal
- 90: erstes Ende
- 92: zweites Ende
- 94: erstes Verbindungselement
- 96: zweites Verbindungselement
- 98: Spalt
- 100: Durchmesser
- 102: Umfangsrand
- 104: Vorsprung
- 106: Höhe
- 108: Umfangswinkel
- 110: Dicke
- 112: erstes Ende
- 114: zweites Ende
- 116: Ausnehmung
- 118: Seiten
- 120: Druckklemmeinrichtung
- 122: Bajonettverbindungseinrichtung
- 124: Nut

## Patentansprüche

1. Gelenkpfanne (14) für eine Gelenkendoprothese (10), insbesondere in Form einer Hüftgelenkpfanne (16) für eine Hüftgelenkendoprothese (12), welche Gelenkpfanne (14) die Form oder im Wesentlichen die Form eines Ausschnitts einer hohlkugeligen Schale (28) aufweist, wobei die Gelenkpfanne eine Pfannenwand (30) umfasst, in welcher Pfannenwand (30) mindestens eine Durchgangsöffnung (38) ausgebildet ist, welche Durchgangsöffnung (38) mit einem Verschlusselement (52) verschlossen ist, welches Verschlusselement (52) einstückig mit der Pfannenwand (30) ausgebildet ist, wobei mindestens eine Sollbruchstelle zwischen dem Verschlusselement (52) und der Pfannenwand (30) ausgebildet ist, welche mindestens eine Sollbruchstelle (70) irreversibel zerstörbar ist zum Entfernen des Verschlusselements (52) aus der Pfannenwand (30), **dadurch gekennzeichnet, dass** die Gelenkpfanne (14) mindestens eine Verbindungseinrichtung (72) umfasst zum kraft- und/oder formschlüssigen Verbinden des aus der Pfannenwand (30) entfernten Verschlusselements (52) mit der Pfannenwand (30) in einer Verbindungsstellung.

2. Gelenkpfanne nach Anspruch 1, **dadurch gekennzeichnet, dass**
a) die Gelenkpfanne (14) eine kugelige oder im Wesentlichen kugelige Außenfläche (34) und eine hohlkugelige oder im Wesentlichen hohlkugelige Innenfläche (36) aufweist
und/oder
b) die mindestens eine Verbindungseinrichtung (72) in Form einer Schraubverbindungseinrichtung (74), einer Bajonettverbindungseinrichtung (122), einer Drehklemmverbindungseinrichtung (120) oder einer Schnappverbindungseinrichtung ausgebildet ist.

3. Gelenkpfanne nach einem der voranstehenden Ansprüche, **dadurch gekennzeichnet, dass** die mindestens eine Verbindungseinrichtung (72) mindestens ein erstes Verbindungselement (94) und mindestens ein zweites Verbindungselement (96) umfasst, dass das mindestens eine erste Verbindungselement (94) an der Pfannenwand (30) angeordnet oder ausgebildet ist, insbesondere an oder im Bereich der Durchgangsöffnung (38), und dass das mindestens eine zweite Verbindungselement (96) am mindestens einen Verschlusselement (52) angeordnet oder ausgebildet ist.

4. Gelenkpfanne nach Anspruch 3, **dadurch gekennzeichnet, dass**
a) das mindestens eine erste Verbindungselement (94) und das mindestens eine zweite Verbindungselement (96) in der Verbindungsstellung kraft- und/oder formschlüssig in Eingriff stehen und in einer Trennstellung, in welcher die mindestens eine Sollbruchstelle (70) irreversibel zerstört ist und das Verschlusselement (52) die mindestens eine Durchgangsöffnung (38) freigibt, außer Eingriff stehen,
und/oder
b) das mindestens eine erste oder zweite Verbindungselement (94, 96) einen Innen- oder Außengewindeabschnitt (78, 76) umfasst
und/oder
c) das mindestens eine erste oder zweite Verbindungselement (94, 96) in Form eines Vorsprungs (104) oder in Form einer zum Vorsprung korrespondierenden Ausnehmung (116) ausgebildet ist.

5. Gelenkpfanne nach einem der voranstehenden Ansprüche, **dadurch gekennzeichnet, dass** die mindestens eine Durchgangsöffnung (38) eine Längsachse (50) definiert, welche in radialer Richtung oder im Wesentlichen in radialer Richtung bezogen auf einen Mittelpunkt (48) der Schale (28) verläuft.

6. Gelenkpfanne nach Anspruch 5, **dadurch gekennzeichnet, dass**
a) der Vorsprung (84; 104) vom mindestens einen Verschlusselement (52) bezogen auf die Längsachse (50) in radialer Richtung weisend abstehend oder von einem die Durchgangsöffnung (38) begrenzenden Rand (68) der Pfannenwand (30) in Richtung auf die Längsachse (50) hin vorstehend angeordnet oder ausgebildet ist
und/oder
b) die zum Vorsprung (104) korrespondierende Ausnehmung (116) in von einem die Durchgangsöffnung (38) begrenzenden Rand (68) der Pfannenwand in Richtung auf die Längsachse (50) hin weisend geöffnet oder vom mindestens einen Verschlusselement (52) bezogen auf die Längsachse (50) in radialer Richtung weisend geöffnet angeordnet oder ausgebildet ist
und/oder
c) die Ausnehmung mindestens einen in Richtung auf die Längsachse (50) hin oder in radialer Richtung von der Längsachse (50) weg weisend geöffneten Nutabschnitt (124) umfasst
und/oder
d) das mindestens eine erste oder zweite Verbindungselement (94, 96) in Form einer zum Vorsprung korrespondierenden Ausnehmung (116) ausgebildet ist und dass die Ausnehmung (116) mindestens abschnittsweise in Richtung auf einen Mittelpunkt (48) der Schale (28) hin weisend geöffnet ist zum Ausbilden einer Einführöffnung für den Vorsprung (104) oder mindestens einen Teil desselben in einer parallel oder im Wesentlichen parallel zur Längsachse (50) verlaufenden Einführrichtung.

7. Gelenkpfanne nach einem der Ansprüche 5 oder 6, **dadurch gekennzeichnet, dass** das mindestens eine erste oder zweite Verbindungselement (94, 96) in Form eines Vorsprungs (104) ausgebildet ist und dass der Innen- oder Außengewindeabschnitt (78, 76) am Vorsprung (84) angeordnet oder ausgebildet ist.

8. Gelenkpfanne nach Anspruch 7, **dadurch gekennzeichnet, dass** der Vorsprung (104) bei unbeschädigter Sollbruchstelle (70) in einer Neutralstellung der mindestens einen Verbindungseinrichtung (72) angeordnet ist, in welcher der Vorsprung (104) in der Einführöffnung oder im Bereich der Einführöffnung angeordnet ist,
wobei insbesondere das Verschlusselement (52) in der Neutralstellung bei irreversibel zerstörter Sollbruchstelle (70) mit der Pfannenwand (30) weder kraft- noch formschlüssig in Eingriff steht.

9. Gelenkpfanne nach einem der voranstehenden Ansprüche, **dadurch gekennzeichnet, dass** die mindestens eine Sollbruchstelle (70) eine Wandstärke (82) aufweist, welche deutlich kleiner ist als eine Dicke (32) der Pfannenwand (30),
wobei insbesondere die Wandstärke (82) weniger als das 0,2-fache der Dicke (32) beträgt, insbesondere weniger als das 0,1-fache.

10. Gelenkpfanne nach einem der Ansprüche 3 bis 9, **dadurch gekennzeichnet, dass** in der Pfannenwand (30) mindestens ein Pulverkanal (88) ausgebildet ist, welcher die Pfannenwand (30) mindestens teilweise durchsetzt und ein erstes offenes Ende (90) und ein zweites offenes Ende (92) aufweist, und dass das erste offene Ende (90) im Bereich des ersten oder zweiten Verbindungselements (94, 96) angeordnet oder ausgebildet ist und dass das zweite offene Ende (92) die Außenfläche (34) oder die Innenfläche (36) der Pfannenwand (30) durchsetzt.

11. Gelenkpfanne nach Anspruch 10, **dadurch gekennzeichnet, dass**
a) ein Durchmesser (100) des mindestens einen Pulverkanals (88) mindestens etwa dem 5-fachen, insbesondere dem 10-fachen, des mittleren Durchmessers der Pulverteilchen entspricht, aus denen die Gelenkpfanne (14) durch ein generatives Fertigungsverfahren ausgebildet ist,
und/oder
b) die Gelenkpfanne eine Mehrzahl von Pulverkanälen (88) umfasst.

12. Gelenkpfanne nach einem der voranstehenden Ansprüche, **dadurch gekennzeichnet, dass**
a) das mindestens eine durch Zerstören der mindestens einen Sollbruchstelle (70) aus der Pfannenwand (30) herausgetrennte Verschlusselement (52) durch Einschrauben um eine von der Durchgangsöffnung (38) definierte Längsachse (50) oder eine Eindrehbewegung um die Längsachse (50) von der Trennstellung in die Verbindungsstellung überführbar ist
und/oder
b) das mindestens eine Verschlusselement (52) mindestens eine Werkzeugelementaufnahme (58) umfasst,
wobei insbesondere die mindestens eine Werkzeugelementaufnahme (58) in Richtung auf den Mittelpunkt (48) der Schale (28) hin weisend geöffnet ist,
und/oder
c) die Gelenkpfanne (14) einstückig, insbesondere monolithisch, ausgebildet ist.

13. Gelenkpfanne nach einem der voranstehenden Ansprüche, **dadurch gekennzeichnet, dass** die Gelenkpfanne (14) durch ein generatives Fertigungsverfahren ausgebildet ist,
wobei insbesondere die Gelenkpfanne (14) durch
a) ein Pulverbettverfahren, durch ein Freiformverfahren, durch ein Flüssigmaterialverfahren oder durch ein anderes Schichtaufbauverfahren ausgebildet ist
und/oder
b) selektives Laserschmelzen, durch selektives Lasersintern, durch selektives Wärmesintern, durch Verfestigen von Pulvermaterial mittels Binder, durch Elektronenstrahlschmelzen, durch Auftragsschweißen, durch ein Metall-Pulver-Auftragsverfahren, durch Kaltgasspritzen, durch Stereolithografie, durch ein Digital-Light-Processing zur Belichtung einsetzendes Verfahren, durch schichtweises Laminieren, durch 3D-Siebdruck von Metallen oder durch lichtgesteuerte elektrophoretische Abscheidung ausgebildet ist.

14. Gelenkpfanne nach einem der voranstehenden Ansprüche, **dadurch gekennzeichnet, dass** die Gelenkpfanne (14) aus einem Metall und/oder aus einem Kunststoff ausgebildet ist,
wobei insbesondere
a) das Metall Titan ist oder enthält
und/oder
b) der Kunststoff Polyetheretherketon ist oder enthält.

15. Gelenkendoprothese (10), insbesondere in Form einer Hüftgelenkendoprothese (12), umfassend eine Gelenkpfanne (14) und einen mit der Gelenkpfanne (14) kugelgelenkig gekoppelten Schaft (18), insbesondere in Form eines Femurschafts (20), **dadurch gekennzeichnet, dass** die Gelenkpfanne (14) in Form einer Gelenkpfanne (14) nach einem der voranstehenden Ansprüche ausgebildet ist,
wobei insbesondere der Schaft (18) einen kugelförmigen oder im Wesentlichen kugelförmigen Gelenkkopf (14) umfasst und dass die Gelenkpfanne (14) einen Gelenkeinsatz (24) mit einer zum Gelenkkopf (22) korrespondierenden Gelenkkopfaufnahme (26) umfasst.

## Claims

1. Joint socket (14) for a joint endoprosthesis (10), in particular in the form of a hip joint socket (16) for a hip joint endoprosthesis (12), which joint socket (14) has the form or substantially the form of a section of a hollow-spherical shell (28), wherein the joint socket comprises a socket wall (30), in which socket wall (30) at least one through-opening (38) is formed, which through-opening (38) is closed with a closure element (52), which closure element (52) is formed as one piece with the socket wall (30), wherein at least one predetermined breaking point is formed between the closure element (52) and the socket wall (30), which at least one predetermined breaking point (70) is irreversibly destroyable for removing the closure element (52) from the socket wall (30), **characterized in that** the joint socket (14) comprises at least one connecting device (72) for force- and/or form-fittingly connecting the closure element (52) removed from the socket wall (30) to the socket wall (30) in a connecting position.

2. Joint socket in accordance with Claim 1, **characterized in that**
a) the joint socket (14) has a spherical or substantially spherical outer surface (34) and a hollow-spherical or substantially hollow-spherical inner surface (36)
and/or
b) the at least one connecting device (72) is configured in the form of a screw connecting device (74), a bayonet connecting device (122), a turning clamp connecting device (120), or a snapping connecting device.

3. Joint socket in accordance with any one of the preceding Claims, **characterized in that** the at least one connecting device (72) comprises at least one first connecting element (94) and at least one second connecting element (96), **in that** the at least one first connecting element (94) is arranged or formed on the socket wall (30), in particular on or in the region of the through-opening (38), and **in that** the at least one second connecting element (96) is arranged or formed on the at least one closure element (52).

4. Joint socket in accordance with Claim 3, **characterized in that**
a) the at least one first connecting element (94) and the at least one second connecting element (96) in the connecting position are in engagement in a force- and/or form-fitting manner and in a separating position, in which the at least one predetermined breaking point (70) is irreversibly destroyed and the closure element (52) unblocks the at least one through-opening (38), are disengaged
and/or
b) the at least one first or second connecting element (94, 96) comprises an internal or external thread section (78, 76)
and/or
c) the at least one first or second connecting element (94, 96) is configured in the form of a projection (104) or in the form of a recess (116) corresponding to the projection.

5. Joint socket in accordance with any one of the preceding Claims, **characterized in that** the at least one through-opening (38) defines a longitudinal axis (50) which runs in radial direction or substantially in radial direction in relation to a center (48) of the shell (28).

6. Joint socket in accordance with Claim 5, **characterized in that**
a) the projection (84; 104) is arranged or formed protruding from the at least one closure element (52) in radial direction in relation to the longitudinal axis (50) or projecting from a rim (68) of the socket wall (30) delimiting the through-opening (38) in the direction toward the longitudinal axis (50)
b) the recess (116) corresponding to the projection (104) is arranged or formed in a rim (68) of the socket wall, which rim (68) delimits the through-opening (38), so as to be open facing in the direction toward the longitudinal axis (50) or open facing from the at least one closure element (52) in radial direction in relation to the longitudinal axis (50)
and/or
c) the recess comprises at least one groove section (124) open facing in the direction toward the longitudinal axis (50) or in radial direction away from the longitudinal axis (50)
d) the at least one first or second connecting element (94, 96) is configured in the form of a recess (116) corresponding to the projection and **in that** the recess (116) is open at least in sections facing in the direction toward a center (48) of the shell (28) for forming an insertion opening for the projection (104) or at least a part thereof in an insertion direction running parallel or substantially parallel to the longitudinal axis (50).

7. Joint socket in accordance with any one of Claims 5 or 6, **characterized in that** the at least one first or second connecting element (94, 96) is configured in the form of a projection (104) and **in that** the internal or external thread section (78, 76) is arranged or formed on the projection (84).

8. Joint socket in accordance with Claim 7, **characterized in that** when the predetermined breaking point (70) is undamaged, the projection (104) is arranged in a neutral position of the at least one connecting device (72) in which the projection (104) is arranged in the insertion opening or in the region of the insertion opening,
wherein, in particular, the closure element (52) in the neutral position with the predetermined breaking point (70) irreversibly broken is neither in engagement in a force- nor a form-fitting manner with the socket wall (30).

9. Joint socket in accordance with any one of the preceding Claims, **characterized in that** the at least one predetermined breaking point (70) has a wall thickness (82) which is significantly smaller than a thickness (32) of the socket wall (30),
wherein, in particular, the wall thickness (82) is less than 0.2 times the thickness (32), in particular less than 0.1 times.

10. Joint socket in accordance with any one of Claims 3 to 9, **characterized in that** in the socket wall (30) is formed at least one powder channel (88) which at least partially passes through the socket wall (30) and has a first open end (90) and a second open end (92), and **in that** the first open end (90) is arranged or formed in the region of the first or second connecting element (94, 96), and **in that** the second open end (92) passes through the outer surface (34) or the inner surface (36) of the socket wall (30).

11. Joint socket in accordance with Claim 10, **characterized in that**
a) a diameter (100) of the at least one powder channel (88) corresponds to at least about 5 times, in particular 10 times, the average diameter of the powder particles from which the joint socket (14) is formed by a generative manufacturing process,
and/or
b) the joint socket comprises a multitude of powder channels (88).

12. Joint socket in accordance with any one of the preceding Claims, **characterized in that**
a) the at least one closure element (52) separated out of the socket wall (30) by destroying the at least one predetermined breaking point (70) is transferable from the separating position into the connecting position by screwing in about a longitudinal axis (50) defined by the through-opening (38) or a rotating-in movement about the longitudinal axis (50)
and/or
b) the at least one closure element (52) comprises at least one tool element receptacle (58),
wherein, in particular, the at least one tool element receptacle (58) is open facing in the direction toward the center (48) of the shell (28),
and/or
c) the joint socket (14) is formed as one piece, in particular monolithically.

13. Joint socket in accordance with any one of the preceding Claims, **characterized in that** the joint socket (14) is formed by a generative manufacturing process,
wherein, in particular, the joint socket (14)
a) is formed by a powder bed process, by a free-forming process, by a liquid material process, or by a different layering process
and/or
b) is formed by selective laser melting, by selective laser sintering, by selective heat sintering, by solidifying powder material by means of binder, by electron beam melting, by deposit welding, by a metal powder deposition process, by cold gas spraying, by stereolithography, by a process using digital light processing for illumination, by lamination by sheets, by 3D screen printing of metals, or by light-controlled electrophoretic deposition.

14. Joint socket in accordance with any one of the preceding Claims, **characterized in that** the joint socket (14) is made of a metal and/or of a plastic,
wherein, in particular,
a) the metal is or contains titanium
and/or
b) the plastic is or contains polyetheretherketone.

15. Joint endoprosthesis (10), in particular in the form of a hip joint endoprosthesis (12), comprising a joint socket (14) and a shank (18), in particular in the form of a femur shank (20), which is coupled to the joint socket (14) in a ball-jointed manner, **characterized in that** the joint socket (14) is configured in the form of a joint socket (14) in accordance with any one of the preceding Claims
wherein, in particular, the shank (18) comprises a ball-shaped or substantially ball-shaped joint head (14), and **in that** the joint socket (14) comprises a joint insert (24) with a joint head receptacle (26) corresponding to the joint head (22).

## Revendications

1. Cavité articulaire (14) pour une endoprothèse articulaire (10), en particulier présentant la forme d'un acétabulum (16) pour une endoprothèse articulaire de hanche (12), laquelle cavité articulaire (14) présente la forme ou sensiblement la forme d'un segment d'une coque sphérique creuse (28), dans laquelle la cavité articulaire comprend une paroi de cavité (30), paroi de cavité (30) dans laquelle au moins une ouverture traversante (38) est réalisée, laquelle ouverture traversante (38) est fermée avec un élément de fermeture (52), lequel élément de fermeture (52) est réalisé d'une seule pièce avec la paroi de cavité (30), dans laquelle au moins un point de rupture est réalisé entre l'élément de fermeture (52) et la paroi de cavité (30), lequel au moins un point de rupture (70) peut être détruit de manière irréversible pour le retrait de l'élément de fermeture (52) de la paroi de cavité (30), **caractérisée en ce que** la cavité articulaire (14) comprend au moins un dispositif de liaison (72) pour la liaison à force et/ou par coopération de formes de l'élément de fermeture (52) retiré de la paroi de cavité (30) à la paroi de cavité (30) dans une position de liaison.

2. Cavité articulaire selon la revendication 1, **caractérisée en ce que**
a) la cavité articulaire (14) présente une surface extérieure (34) sphérique ou sensiblement sphérique et une surface intérieure (36) sphérique creuse ou sensiblement sphérique creuse
et/ou
b) le au moins un dispositif de liaison (72) est réalisé sous la forme d'un dispositif de liaison à vis (74), d'un dispositif de liaison à baïonnette (122), d'un dispositif de liaison à serrage rotatif (120) ou d'un dispositif de liaison par encliquetage.

3. Cavité articulaire selon l'une quelconque des revendications précédentes, **caractérisée en ce que** le au moins un dispositif de liaison (72) comprend au moins un premier élément de liaison (94) et au moins un deuxième élément de liaison (96), que le au moins un premier élément de liaison (94) est disposé ou réalisé sur la paroi de cavité (30), en particulier sur ou dans la zone de l'ouverture traversante (38), et que le au moins un deuxième élément de liaison (96) est disposé ou réalisé sur le au moins un élément de fermeture (52).

4. Cavité articulaire selon la revendication 3, **caractérisée en ce que**
a) le au moins un premier élément de liaison (94) et le au moins un deuxième élément de liaison (96), dans la position de liaison, sont en prise à force et/ou par coopération de formes et, dans une position de séparation, dans laquelle le au moins un point de rupture (70) est détruit de manière irréversible et l'élément de fermeture (52) libère la au moins une ouverture traversante (38), ne sont pas en contact,
et/ou
b) le au moins un premier ou deuxième élément de liaison (94, 96) comprend une partie filetage femelle ou mâle (78, 76)
et/ou
c) le au moins un premier ou deuxième élément de liaison (94, 96) est réalisé sous forme d'une partie saillante (104) ou sous forme d'un évidement (116) correspondant à la partie saillante.

5. Cavité articulaire selon l'une quelconque des revendications précédentes, **caractérisée en ce que** la au moins une ouverture traversante (38) définit un axe longitudinal (50), lequel s'étend dans la direction radiale ou sensiblement dans la direction radiale par rapport à un centre (48) de la coque (28).

6. Cavité articulaire selon la revendication 5, **caractérisée en ce que**
a) la partie saillante (84 ; 104) est disposée ou réalisée de manière à faire saillie d'au moins un élément de fermeture (52) par rapport à l'axe longitudinal (50) de manière orientée dans la direction radiale ou de manière à faire saillie d'un bord (68) de la paroi de cavité (30), délimitant l'ouverture traversante (38), en direction de l'axe longitudinal (50)
et/ou
b) l'évidement (116) correspondant à la partie saillante (104) est ouvert de manière orientée d'un bord (68), délimitant l'ouverture traversante (38), de la paroi de cavité en direction de l'axe longitudinal (50) ou placé ou réalisé en étant ouvert de manière orientée de l'au moins un élément de fermeture (52) par rapport à l'axe longitudinal (50) dans la direction radiale
et/ou
c) l'évidement comprend au moins une partie de rainure (124) ouverte de manière orientée en direction de l'axe longitudinal (50) ou en s'écartant de l'axe longitudinal (50) dans la direction radiale
et/ou
d) le au moins un premier ou deuxième élément de liaison (94, 96) est réalisé sous forme d'un évidement (116) correspondant à la partie saillante et que l'évidement (116) est ouvert au moins sur certaines sections en étant orienté en direction d'un centre (48) de la coque (28) pour la réalisation d'une ouverture d'introduction pour la partie saillante (104) ou au moins une partie de celle-ci dans une direction d'introduction s'étendant parallèlement ou sensiblement parallèlement à l'axe longitudinal (50).

7. Cavité articulaire selon l'une quelconque des revendications 5 ou 6, **caractérisée en ce que** le au moins un premier ou deuxième élément de liaison (94, 96) est réalisé sous forme d'une partie saillante (104) et que la section de filetage femelle ou mâle (78, 76) est disposée ou réalisée sur la partie saillante (84).

8. Cavité articulaire selon la revendication 7, **caractérisée en ce que** la partie saillante (104), lorsque le point de rupture (70) est intact, est disposée dans une position neutre de le au moins un dispositif de liaison (72), dans laquelle la partie saillante (104) est disposée dans l'ouverture d'introduction ou dans la zone de l'ouverture d'introduction,
dans laquelle en particulier l'élément de fermeture (52), dans la position neutre, lorsque le point de rupture (70) est détruit de manière irréversible, n'est en contact ni à force ni par coopération de formes avec la paroi de cavité (30).

9. Cavité articulaire selon l'une quelconque des revendications précédentes, **caractérisée en ce que** le au moins un point de rupture (70) présente une épaisseur de paroi (82), laquelle est nettement inférieure à une épaisseur (32) de la paroi de cavité (30),
dans laquelle en particulier l'épaisseur de paroi (82) est inférieure au moins à 0,2 fois l'épaisseur (32), en particulier inférieure à 0,1 fois.

10. Cavité articulaire selon l'une quelconque des revendications 3 à 9, **caractérisée en ce qu'**au moins un canal de poudre (88), lequel traverse au moins en partie la paroi de cavité (30) et présente une première extrémité ouverte (90) et une deuxième extrémité ouverte (92), est réalisé dans la paroi de cavité (30), et que la première extrémité ouverte (90) est disposée ou réalisée dans la zone du premier ou deuxième élément de liaison (94, 96) et que la deuxième extrémité ouverte (92) traverse la surface extérieure (34) ou la surface intérieure (36) de la paroi de cavité (30).

11. Cavité articulaire selon la revendication 10, **caractérisée en ce que**
a) un diamètre (100) du au moins un canal de poudre (88) correspond au moins approximativement à 5 fois, en particulier à 10 fois le diamètre moyen des particules de poudre à partir desquelles la cavité articulaire (14) est réalisée par un procédé de fabrication additive,
et/ou
b) la cavité articulaire comprend une pluralité de canaux de poudre (88).

12. Cavité articulaire selon l'une quelconque des revendications précédentes, **caractérisée en ce que**
a) le au moins un élément de fermeture (52) détaché de la paroi de cavité (30) par destruction de le au moins un point de rupture (70) peut être amené de la position de séparation dans la position de liaison par vissage autour d'un axe longitudinal (50) défini par l'ouverture traversante (38) ou un mouvement de rotation intérieure autour de l'axe longitudinal (50)
et/ou
b) le au moins un élément de fermeture (52) comprend au moins un logement d'élément d'outil (58),
dans laquelle en particulier le au moins un logement d'élément d'outil (58) est ouvert de manière orientée en direction du centre (48) de la coque (28),
et/ou
c) la cavité articulaire (14) est réalisée d'une seule pièce, en particulier de façon monolithique.

13. Cavité articulaire selon l'une quelconque des revendications précédentes, **caractérisée en ce que** la cavité articulaire (14) est réalisée par un procédé de fabrication additive,
dans laquelle en particulier la cavité articulaire (14) est réalisée par
a) un procédé sur lit de poudre, par un procédé de formage libre, par un procédé à matériau liquide ou par un autre procédé de constitution par couches
et/ou
b) fusion sélective par laser, par frittage laser sélectif, par frittage thermique sélectif, par solidification de matériau pulvérulent au moyen de liants, par fusion par faisceaux d'électrons, par rechargement dur, par un procédé d'application de poudre métallique, par projection à froid, par stéréolithographie, par un procédé utilisant un traitement numérique de la lumière pour l'exposition, par stratification par couches, par sérigraphie 3D de métaux ou par dépôt par électrophorèse commandé par la lumière.

14. Cavité articulaire selon l'une quelconque des revendications précédentes, **caractérisée en ce que** la cavité articulaire (14) est réalisée à partir d'un métal et/ou à partir d'un plastique,
dans laquelle en particulier
a) le métal est ou contient du titane
et/ou
b) le plastique est ou contient du polyétheréthercétone.

15. Endoprothèse articulaire (10), en particulier sous forme d'une endoprothèse articulaire de hanche (12), comprenant une cavité articulaire (14) et une tige (18) accouplée par une articulation à rotule à la cavité articulaire (14), en particulier sous forme d'une tige fémorale (20), **caractérisée en ce que** la cavité articulaire (14) est réalisée sous forme d'une cavité articulaire (14) selon l'une quelconque des revendications précédentes,
dans laquelle en particulier la tige (18) comprend une tête d'articulation (14) sphérique ou sensiblement sphérique et que la cavité articulaire (14) comprend un insert d'articulation (24) avec un logement de tête d'articulation (26) correspondant à la tête d'articulation (22).
